# EUROPEAN PATENT SPECIFICATION

(11) **EP 4 429 560 B1**
(45) Date of publication and mention of the grant of the patent: **30.07.2025**
(21) Application number: 22813920.0
(22) Date of filing: 03.11.2022
(51) Int. Cl.: A61B 8/08, A61B 8/00, A61B 34/30, A61B 34/00

(54) **CONTROLLER FOR IMAGING AND CONTROLLING ROBOTIC DEVICE USING IMAGING INTERFACE**
STEUERGERÄT ZUR ABBILDUNG UND STEUERUNG EINER ROBOTERVORRICHTUNG UNTER VERWENDUNG EINER ABBILDUNGSSCHNITTSTELLE
ORGANE DE COMMANDE POUR IMAGER ET COMMANDER UN DISPOSITIF ROBOTIQUEAU MOYEN D'UNE INTERFACE D'IMAGERIE

(30) Priority: 10.11.2021 US 202163277731 P; 04.02.2022 EP 22155097
(43) Date of publication of application: 18.09.2024
(73) Proprietor: Koninklijke Philips N.V., 5656 AG Eindhoven (NL)
(72) Inventor: BALICKI, Marcin A., 5656 AG Eindhoven (NL)
(74) Representative: Philips Intellectual Property & Standards
(86) International application number: PCT/EP2022/080629
(87) International publication number: WO 2023/083676

(56) References cited:
- WO-A1-2019/050821
- WO-A1-2019/204699
- US-A1- 2012 265 071
- US-A1- 2021 322 106

## Description

### BACKGROUND

Complex interventional procedures, such as peripheral vascular interventions, use ultrasound to provide imaging feedback when controlling movement of an interventional device maneuvered within an anatomical structure of a subject (patient). Such procedures require involvement of at least two clinicians (users) to coordinate the imaging and robotic device manipulation. One user (e.g., ultrasound technician) manipulates an ultrasound probe to obtain images of the anatomy of interest, typically under the direction of the other user (e.g., physician), while the other user interprets the images and simultaneously manipulates the interventional device with their hands or via a robotic interface.

This is a common problem in endovascular interventions, for example, where the interventional device comprises a catheter and a guidewire that are operated using both hands and are manipulated at access site (e.g., groin) which is often far from the treatment site (e.g., lower leg). Again, additional skilled user is needed to manipulate and hold the ultrasound probe to image the anatomy and the catheter and guidewire positioned in the anatomy. Similarly, percutaneous interventions and minimally invasive interventions, such as such as ablations and atherectomies, in which multiple devices must be controlled. Accordingly, there is a need for enabling consolidated control of interventional and imaging devices, such that a single user is able to seamlessly operate both during the interventional procedure.

US 2012/265071 A1 discloses a controller for controlling a robotic device configured for insertion into an anatomical structure of a subject, the controller comprising:
a. at least one processor; and
b. a non-transitory memory for storing machine executable instructions that, when executed by the at least one processor, cause the at least one processor to:
   - receive ultrasound image data from an imaging interface device, the ultrasound image data corresponding to an ultrasound image showing the anatomical structure and a portion of the robotic device within in the anatomical structure, wherein the imaging interface device is maneuverable for providing the ultrasound image data;
   - detect a movement, or receive an information representative of the detected movement of the imaging interface device using a navigation system employing optically or electromagnetically detectable markers or an RFID technology; and
   - output at least one command for controlling movement of the robotic device from a first position to a second position in response to the detected movement of the imaging interface device.

### SUMMARY

According to an aspect of the present disclosure, a controller according to claim 1 is provided**.**

According to another aspect of the present disclosure, a non-transitory computer readable medium according to claim 15 is provided.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 is a simplified block diagram of a system for controlling imaging and a robotic device insertable into an anatomical structure of a subject, according to a representative embodiment.
FIG. 2A is a perspective view of an ultrasound probe with a push button actuator for selecting imaging and control modes, according to representative embodiments.
FIG. 2B is a perspective view of an ultrasound probe with a slider button actuator for selecting imaging and control modes, according to representative embodiments.
FIG. 2C is a perspective view of an ultrasound probe with combination push and slider buttons actuator for selecting imaging and control modes, according to representative embodiments.
FIG. 3 shows a perspective view of an imaging interface translated while imaging an anatomical structure, according to a representative embodiment.
FIG. 4 shows a perspective view of an imaging interface rotated while imaging an anatomical structure, according to a representative embodiment.
FIG. 5 is a simplified block diagram of a system controller for controlling imaging and a robotic device insertable into an anatomical structure of a subject, according to a representative embodiment.
FIG. 6 is a flow diagram of a method for controlling imaging and a robotic device insertable into an anatomical structure of a subject, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

The various embodiments described herein streamline control of imaging and interventional devices using an imaging interface device operable by a single user. That is, the imaging interface device enables the single user to directly control the imaging modality, while simultaneously controlling parameters of the imaging modality, or other devices in the system, such as a robotic device inside the subject during an interventional procedure by manipulating the imaging interface device. The imaging interface device reduces redundant communication, is intuitive, and minimizes or potentially eliminates the need for a supporting imaging modality technician.

Generally, a system controller is configured to control at least one control parameter of a robotic device having at least one degree of freedom (DOF) of control in response to motion of an imaging interface device (e.g., ultrasound probe) held by the user. Operation of the imaging interface device enables command of a robot controller to follow the motion of the imaging interface device, where the motion is derived from the content in the images, which are also provided through imaging interface device. Thus, the imaging interface device itself is effectively utilized as a joystick to allow the user to control robotic device and any interventional devices operable via the robotic device, such as endovascular devices, for example, that are inside the subject and being tracked in the images (e.g., ultrasound volume). So, the user may image the anatomy of interest, and when motion of the robotic device is desired (e.g., advancing a guidewire inside a vessel), the user may enter the control (joystick) mode through operation of an actuator on the imaging interface device. Alternatively, or in addition, the system controller may be configured to control at least one control parameter of the imaging modality itself, such as frequency, pulse wave, color, and power, for example, in response to motion of the imaging interface device.

With regard to controlling a robotic device, the system controller takes a note of the position of a trackable feature (e.g., distal tip) of the robotic device in the imaging volume and creates a reference plane relative to the imaging interface device (and inherently the imaging volume) that is perpendicular to a longitudinal axis of the robotic device. When the user translates the imaging interface device along the direction of the robotic device, for example, the distance of the trackable feature of the robotic device to the reference plane is changing. The system controller uses this distance as an error input to servo (e.g., using PID servo control loop) the robotic device relative to the reference plane, such that the robotic device will appear to travel with the image. The servo motion continues until a tolerance error is satisfied. When the user rotates the imaging interface device, either during translation or not, the amount and direction of the rotation is detected through direct image processing or (not according to the claimed invention) positional data of the imaging interface device, e.g., using an onboard Inertial Measurement Unit (IMU), and the system controller causes the robotic device to rotate in a corresponding fashion. The user may then leave the control mode and return to the imaging mode when the actuator on the imaging interface device is inactivated.

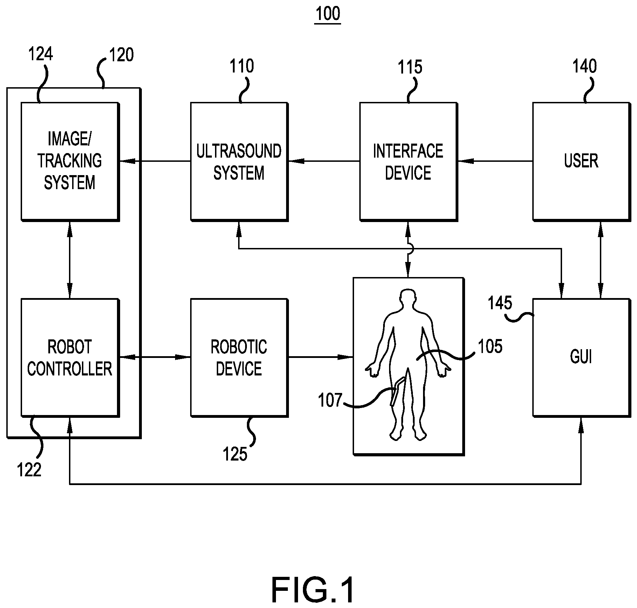

FIG. 1 is a simplified block diagram of a system for controlling imaging and a robotic device insertable into an anatomical structure of a subject, according to a representative embodiment.

Referring to FIG. 1, system 100 is configured to control placement and operation of a robotic device 125 inserted in an anatomical structure 107 of a subject (patient) 105 under direction of user (clinician) 140 during an interventional procedure. The anatomical structure 107 may be a vascular structure (e.g., artery), and the robotic device 125 may include or hold a catheter and/or a guidewire, for example, inserted into the vascular structure. The robotic device 125 may include or hold various other interventional devices, such as surgical devices (e.g., loop cutters, clamps), ablation devices, imaging and sensing devices, or the like, without departing from the scope of the present teachings.

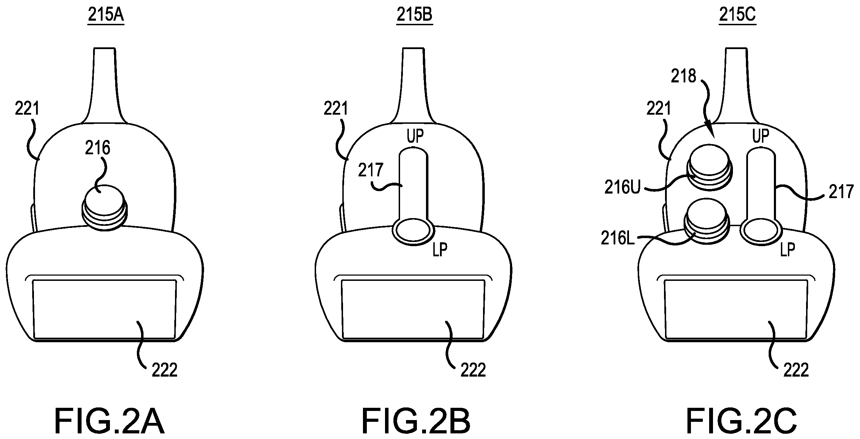

In the depicted embodiment, the system 100 includes an ultrasound system 110 and a system controller 120, which includes a robot controller 122, and an image processing/device tracking system 124. The ultrasound system 110 system is configured to receive ultrasound image data of the subject 105 through an imaging interface device 115. The imaging interface device 115 may be a handheld ultrasound probe, for example, being manipulated by the user 140 while viewing a graphical user interface (GUI) 145 to obtain desired views of the anatomical structure 107 and/or the robotic device 125 while inside the anatomical structure 107. Examples of the imaging interface device 115 as an ultrasound probe are shown in FIGs. 2A, 2B and 2C, discussed below. In various embodiments, the imaging interface device 115 may be implemented by any other type of compatible interfaces, such as foot pedals, wheels and knobs, for example, in addition to or in place of an ultrasound probe.

When the imaging interface device 115 is implemented as an ultrasound probe, it may include multiple ultrasound transducers in a transducer array configured to emit acoustic waves into the subject 105, and detecting elements in a detecting array configured to receive corresponding echoes under control of the ultrasound system 110 to provide ultrasound image data that includes imaging of the anatomical structure as well as the robotic device 125 inside the anatomical structure. The ultrasound system 110 provides the ultrasound image data to the GUI 145 to be viewed by the user 140.

The system controller 120 is operable in two selectable modes, which may be selected by the user 140 through the imaging interface device 115, for example, during the interventional procedure. As discussed in more detail below, the two selectable modes include an imaging (first) mode and a control (second) mode. In the imaging mode, the system controller 120 receives from the imaging interface device 115 the ultrasound image data of the anatomical structure 107 and the robotic device 125 in the anatomical structure 106. In the control mode, the system controller 120 receives movement information from imaging interface device 115 for controlling movement of the robotic device 125 in the anatomical structure 107. The movement information may include translational movement of the imaging interface device 115 across the surface of the subject 105 in a direction the user wishes to guide the robotic device 125, and rotational movement of the imaging interface device 115 on the surface of the subject 105 at an angle the user wishes to rotate the robotic device 125. The imaging interface device 115 may be configured to enable easy selection of the imaging mode and the control mode by the user while manipulating the imaging interface device 115, as discussed below.

The robot controller 122 controls movement of the robotic device 125 within the anatomical structure 107 during the interventional procedure. Movement of the robotic device 125 includes translational movement for advancing and retracting the robotic device 125 into and out of anatomical structure 107, rotational movement for rotating the robotic device 125 within anatomical structure 106, e.g., in accordance with the available degrees of freedom (DOF) of the robotic device 125, articulation movement for bending or articulating the robotic device 125. The robotic device 125 may also control movement and operation of an active component, such as a laser. The robot controller 122 may control movement of the robotic device 125 through operation of servo motors (servos) integrated with the robotic device 125, as is well known in the art. Thus, the movement may be referred to as servoing the robotic device 125. The robot controller 122 may be implemented as a proportional-integral-derivative (PID) controller, for example.

The robot controller 122 and the robotic device 125 may have autonomous capability, in which case the user 140 may control a subset of actuated degrees of freedom. For example, the user 140 may control articulation or directional movement of the robotic device 125 by manipulation of the imaging interface device 115, while the robot controller 122 advances (translates) the robotic device 125 at a constant rate. Likewise, the user 140 may control the amount of advancement or retraction of the robotic device 125 within the anatomical structure 107 using translation input, while the robot controller 122 simultaneously centers the robotic device 125 in the middle of the anatomical structure 107 cross-section (e.g., in robotic device 125, which may be magnetically actuated).

The image processing/device tracking system 124 receives the ultrasound image data from the ultrasound system 110, identifies movements of the imaging interface device 115 from the ultrasound image data relative to tracked anatomical features in the image data, such as speckle, vessels, for example, identifies the image of the robotic device 125 from the ultrasound image data, and calculates movements of the robotic device 125 responsive to the movements of the imaging interface device 115.

As mentioned above, the imaging interface device 115 is configured to operate in an imaging mode and a control mode based on selection by the user while operating the imaging interface. In the imaging mode, the imaging interface device 115 outputs the ultrasound image data of the anatomical structure 107 and the robotic device 125 in the anatomical structure 107 to the ultrasound system 110. The ultrasound image data is obtained in a known manner, for example, by the user 140 manually manipulating pressure and direction of the imaging interface device 115 (e.g., ultrasound probe) on the surface of the subject 105 while viewing corresponding ultrasound images on the GUI 145 in order to capture desired views. In alternative configurations, the imaging interface device 115 may be manipulated automatically or partially automatically using robotic control, without departing from the scope of the present teaches. In the control mode, the imaging interface device 115 controls movement of the robotic device 125. That is, translational movements of the imaging interface device 115 across the surface of the subject 105 cause corresponding translational movements of the robotic device 125. Likewise, rotational movements of the imaging interface device 115 cause corresponding rotational movements of the robotic device 125. With regard to the rotational movements, in particular, the imaging interface device 115 effectively acts as a joy stick operable by the user 140 when in the control mode.

Notably, the user 140 is able to conveniently select the imaging and control modes using the imaging interface device 115, while holding the imaging interface device 115 during the interventional procedure. For example, when the imaging interface device 115 is an ultrasound probe, the imaging interface device 115 includes a probe handle configured to be grasped by the user 140 for performing both ultrasound imaging and robotic device control without the need of an additional person for performing one or the other of the functions. The imaging interface device 115 includes an actuator, such as a push button or slider button, configured to be operated manually by the user 140 while holding the imaging interface device 115. Again, when the imaging interface device 115 is an ultrasound probe, the actuator may be integrated in the probe handle, so that the actuator is easily accessed and operated by the user while grasping the probe handle to selectively activate the imaging mode and the control mode.

In an embodiment, a virtual probe representing the imaging interface device 115 may be rendered on the GUI 145 and/or on augmented or virtual reality displays (not shown). The GUI 145 enables multiple simultaneous inputs, with regard to selection of the imaging mode and the control mode, as well as other functionality, and may display different scales and values. In another embodiment, the imaging interface device 115 may be controlled by the user telerobotically, as opposed to being handheld. In this case, the user 140 may select the imaging and control modes by remote control.

FIGs. 2A, 2B and 2C show perspective views of ultrasound probes with integrated actuators for selecting imaging and control modes, according to representative embodiments.

Referring to FIG. 2A, ultrasound probe 215A includes a push button 216 as the integrated actuator for selecting the imaging mode and the control mode. The ultrasound probe 215A includes a probe handle 221 for the user 140 to manipulate the ultrasound probe 215A, and a transducer/detector array 222 for transmitting acoustic waves into the subject 105, and receiving corresponding echoes to provide the ultrasound image data. The push button 216 is configured so that, when grasping the probe handle 221 with their hand, the user 140 is able to easily depress the push button 216 in order to switch between the imaging mode and the control mode using the same hand while maintaining the grasp on the probe handle 221. The push button 216 may be actuated using one or more fingers, thumb or other portions of the user's hand.

In an embodiment, ultrasound probe 215A enters the imaging mode when push button 216 is depressed, and enters the control mode when the push button 216 is released, or vice versa. Alternatively, the push button 216 may have one position, and simply switch back and forth between the imaging mode and the control mode each time the push button 216 is pressed. **In** this configuration, the push button 216 may simply be a touch sensitive button instead of a physically depressible push button.

Referring to FIG. 2B, ultrasound probe 215B includes a slider button 217 on the probe handle 221 as the integrated actuator for selecting the imaging mode and the control mode. The slider button 217 is configured so that, when grasping the probe handle 221, the user is able to easily slide the slider button 217 between an upper position UP and a lower position LP in order to switch between the imaging mode and the control mode using the same hand while maintaining the grasp on the probe handle 221. In an embodiment, ultrasound probe 215A enters the imaging mode when slider button 217 is in the upper position UP, and enters the control mode when the slider button 217 is in the lower position LP, or vice versa. The slider button 217 may be actuated by a sliding movement or a scrolling movement, like a scroll wheel, using one or more fingers, thumb or other portions of the user's hand.

Referring to FIG. 2C, ultrasound probe 215C includes a combination controller 218 on the probe handle 221 as the integrated actuator for selecting the imaging mode and the control mode. In the depicted embodiment, the combination controller includes upper push button 216U and lower push button 216L, which function as described above with reference to the push button 216, and slider button 217 so that, when grasping the probe handle 221, the user is able to easily operating either or all of the upper and lower push buttons 216U and 216L and the slider button 217, as described above. Having both the upper and lower push buttons 216U and 216L enables choosing different parameters to be controlled by the motion of the ultrasound probe 215C, independently or simultaneously. Examples include robotic device translation, robotic device rotation, ultrasound imaging parameters, treatment device actuations, such as laser power in laser atherectomy. The combination controller 218 may be actuated using one or more fingers, thumb or other portions of the user's hand.

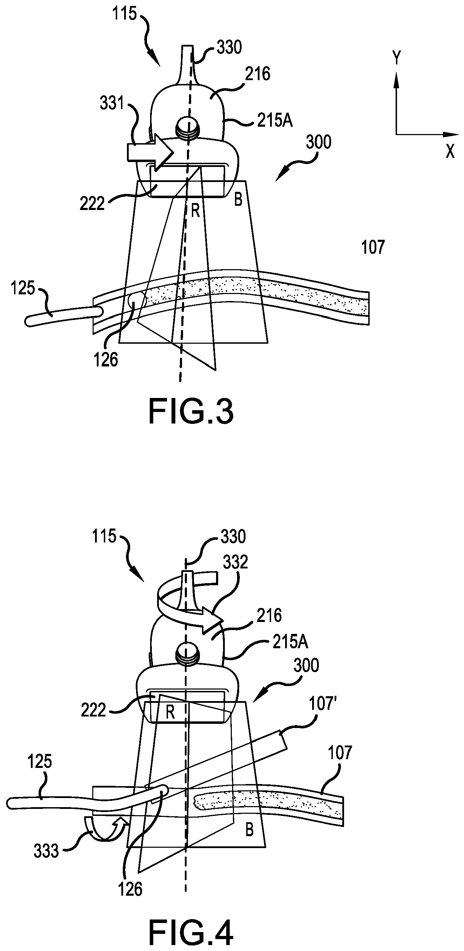

FIG. 3 shows a perspective view of an imaging interface translated while imaging an anatomical structure, according to a representative embodiment.

Referring to FIG. 3, imaging interface device 115 (e.g., ultrasound probe 215A) is shown imaging the anatomical structure 107 and robotic device 125 by emitting acoustic waves 300 into the subject 105 and receiving corresponding echo signals (not shown). The imaging interface device 115 defines an image plane B in ultrasound space that, for purposes of illustration, extends from the transducer/detector array 222 to include the longitudinal axis of the robotic device 125 and/or the longitudinal section of the anatomical structure. That is, portions of the robotic device 125 located inside the anatomical structure 107 along the longitudinal axis are in the image plane B, in both the imaging mode and the control mode of the imaging interface device 115. In the case of a matrix ultrasound probe, such imaging planes can be constructed using beam forming techniques or slicing of 3D volumetric images, both known in the art. In some cases, the image plane B may be located elsewhere relative to the imaging interface device 115, e.g., parallel to longitudinal axis 330 and extending from a line on the transducer/ detector array, and/or may be designated by the user 140, without departing from the scope of the present teachings.

In addition, a virtual reference plane R is defined in ultrasound space relative to the transducer/detector array 222, e.g., whenever the push button 216 is actuated in a manner causing the imaging interface device 115 to enter the control mode. The reference plane R intersects the image plane B (e.g., at about a 90 degree angle) at a first position of a trackable feature of the robotic device 125 being tracked. The reference plane R is substantially perpendicular to a longitudinal axis of the robotic device 125. In the depicted example, the trackable feature of the robotic device 125 being tracked is the distal tip 126 of the robotic device 125. However, the trackable feature is not limited to the distal tip 126, and may be another predetermined section of the robotic device 125, a marker on the robotic device 125, a bend in the robotic device 125, or a predetermined distance from one of the distal tip 126, the other predetermined section, the marker, or the bend, for example.

The control mode is selected to translate the trackable feature of the robotic device from the first position to a second position. In the control mode, a translational movement of the imaging interface device 115 is detected by the image processing/device tracking system 124 when the imaging interface device 115 is translated on the surface of the subject 105 relative to the anatomical structure 107. The direction, distance and/or velocity of the image interface translation may be supplemented by data provided by IMUs on the imaging interface device 115.

The reference plane R is defined relative to the imaging interface device 115, thus moving the imaging interface device 115 moves the reference plane R relative to the anatomy. In particular, when the imaging interface device 115 is translated in the x direction as shown by arrow 331, the reference plane R implicitly follows the translation, moving along the image plane B in the same direction by the same distance. In an embodiment, the translation of the imaging interface device 115 controls force input that is applied to the robotic device 125 towards the reference plane R.

Therefore, to move the robotic device 125 by maneuvering the imaging interface device 115, the user 140 initially depresses the push button 216 to enter the control mode. In the control mode, the image processing/device tracking system 124 locates the trackable feature (e.g., the distal tip 126) of the robotic device 125 in the ultrasound image provided by the ultrasound system 110 in the imaging mode. The image processing/device tracking system 124 may locate the robotic device 125 and its distal tip 126, as well as the anatomical structure 107, using artificial intelligence (AI) segmentation, for example, in which case the image plane B is created from the anatomical structure 107. The image processing/device tracking system 124 creates the image plane B extending from the imaging interface device 115, and further creates the reference plane R intersecting the image plane B at a first position of the trackable feature. This first reference plane R provides a reference datum relative to the imaging interface device 115 based on the first position of the trackable feature in the image plane B. The reference datum may be displayed on the GUI 145, along with the ultrasound image of the anatomical structure 107 and the robotic device 125.

Then, when the user 140 again activates (or continues to activate) the push button 216 to enter the control mode, the imaging interface device 115 can be used, e.g., as a joystick, to control movement of the robotic device 125. In FIG. 3, the user 140 translates the imaging interface device 115 across the surface of the subject 105 in the x direction indicated by the arrow 331 from a first location to a new second location. In response, the information from image processing/device tracking system 124 is used by robot controller 122 to set a new image plane B on the ultrasound system 110 in association with the movement of the imaging interface device 115 to the second location. The image plane B is defined so that tip and the distal longitudinal section of the robotic device 125 contained in the image plane B (in case there is inadvertent motion out of original image plane B). The imaging interface device 115 continues to collect images in the reference plane R, which is defined relative to the imaging interface device 115 probe such that it images in the same location relative to the imaging interface device 115 regardless of image content or tracking information.

The robot controller 122 receives information from the imaging processing/device tracking system 124 identifying the position of the robotic device 125 in the ultrasound image. The robot controller 122 determines positions of the trackable feature of the robotic device 125 in the image plane B. The robot controller 122 determines a distance within the image plane B between the current trackable feature of robotic device 125 and the intersection between the reference plane R and the image plane B, and servos the robotic device 125 so that the trackable feature of the robotic device 125 moves toward this intersection. The robot controller 122 then receives additional information from the imaging processing/device tracking system 124 identifying the next position of the robotic device 125 in the ultrasound image, and repeats the subsequent steps in a loop until a threshold is met.

In an embodiment, the robot controller 122 uses the determined distance as a position error, and translates the robotic device 125 toward the location of the reference plane R by correcting for the position error. In other words, as the user 140 translates the imaging interface device 115 in the direction x, the distance of the trackable feature of the robotic device 125 from the first position relative to the anatomy to the reference plane R increases. So, the robot controller 122 uses this distance as an error input to servo the robotic device 125 towards the reference plane R, for example, using a PID servo control loop, until the second position associated is aligned with the reference plane R. That is, the robot controller 122 continues the servo motion until a tolerance error is satisfied. The imaging interface device 115 may continue to provide ultrasound images during the translation, in which case the movement of the robotic device 125 is captured and displayed on the GUI 145 in real time. Thus, the robotic device 125 moving through the anatomical structure 107 will appear to travel with the ultrasound image.

The user 140 leaves the control mode and re-enters the imaging mode when the push button 216 is inactivated (e.g., released or depressed again). Then, the user 140 may again manipulate the imaging interface device 115 at the second location to obtain ultrasound images of the anatomical structure 107 and the robotic device 125, which are displayed on the GUI 145. This assumes that imaging is the default mode, which recurs when the push button 216 is not pressed. Alternatively, the button press may act as a toggle, it which case the push button 216 will need to be pressed again to disengage modes.

FIG. 4 shows a perspective view of an imaging interface rotated while imaging an anatomical structure, according to a representative embodiment.

Referring to FIG. 4, imaging interface device 115 (e.g., ultrasound probe 215A), for example, is shown imaging the anatomical structure 107 and robotic device 125 by emitting acoustic waves 300 into the subject 105 and receiving corresponding echo signals (not shown). With regard to rotation of the imaging interface device 115, the user 140 initially depresses the push button 216, in response to which the imaging interface device 115 enters the control mode. In the control mode, the image processing/device tracking system 124 locates the trackable feature (e.g., the distal tip 126) of the robotic device 125 in the ultrasound image provided by the ultrasound system 110 in the imaging mode. The image processing/device tracking system 124 may locate the robotic device 125 and its distal tip 126, as well as the anatomical structure 107, using AI segmentation, for example. The image processing/device tracking system 124 creates the image plane B extending from the imaging interface device 115, and further creates the reference plane R intersecting the longitudinal axis 330 and an initial angle (e.g., 90 degrees to plane B), where plane R is virtually attached to one or more tracked anatomical features. The orientation of the reference plane R may provide a first reference image relative to the imaging interface device 115 and the image plane B, which may be displayed on the GUI 145.

When the user 140 rotates the imaging interface device 115 while holding down the push button 216, the imaging interface device 115 can be used, e.g., as a joystick, to control rotation of the robotic device 125, indicated by arrow 332. In some embodiments, consecutive button presses of the push button 216 may be used for clutching in and out. In the depicted example, the objective of rotating the imaging interface device 115 is to control directional movement (e.g., rotation about the longitudinal axis or articulation of the distal tip 126) of the robotic device 125, indicated by arrow 333 in FIG. 4, for steering the robotic device 125 to a branch 107' of the anatomical structure 107. The magnitude of the rotation or articulation of the device is proportional to the rotational deviation of the probe relative to the reference frame R.

Alternatively, in a similar control fashion as above, during a particular control mode, the user 140 rotates the imaging interface device 115 to define the orientation of the reference plane R, which is virtually anchored to anatomical features in the images. Once the control mode is exited, the robot controller 122 uses the distance metric from the distal tip 126 of the robotic device 125 to the reference plane R (from the image processing) to actuate the robotic device 125 to move (e.g., rotate or articulate) towards the reference plane R location relative to the anatomy. Meanwhile, the robot controller 122 receives information from the imaging processing/device tracking system 124 identifying the new orientation the reference plane R and the corresponding angle of rotation, either during the rotation maneuver or after the rotation maneuver is complete. The robot controller 122 servos the robotic device 125 to move (e.g., articulate) by the angle of rotation relative to the image plane B, e.g., steering the distal tip 126 of the robotic device 125 from a main portion of the anatomical structure 107 into the branch 107'. The imaging interface device 115 may continue to provide ultrasound images during the translation, such that the movement of the robotic device 125 is captured and displayed on the GUI 145 along with the rotation of the ultrasound probe 215A.

The GUI 145 may display the basic three-dimensional volume or, in the case of two-dimensional multiple plane imaging, the GUI 145 may display the image plane B in a static position and show reference plane R dynamically relative to plane B, in a 3D perspectives, as shown in FIG. 4. That is, since this is essentially beam steering of the image plane B, the display of the GUI 145 is able to stay on the target region.

The amount and direction of rotation may be detected by direct image observation by the image processing/device tracking system 124 with image processing, such as speckle tracking, for example. The amount and direction of rotation may also be detected or supplemented from data provided by the IMUs on the imaging interface device 115. The amount and direction of rotation relative to the first orientation of the reference plane R as determined by the image processing/device tracking system 124 is input to the robot controller 122. Other compatible rotation detection techniques may be incorporated without departing from the scope of the present teachings.

The robot controller 122 may also apply one or more boundary conditions linked to physical attributes of the anatomical structure 107, such as vessel walls, limiting the translational and/or rotational movements. For example, the one or more boundary conditions may be defined by an anatomical model and/or pre-op images of the anatomical structure, where physical boundaries are identified either automatically using AI segmentation of an ultrasound image, for example, or manually through user 140 interaction the anatomical model and/or the pre-op images via the GUI 145. The boundary conditions are then implemented to avoid contact between the robotic device 125 and the anatomical structure 107. For example, the boundary conditions may cause movement of the robotic device 125 associated with the translation or rotation of imaging interface device 115 to stop when the tracking feature or other portion of the robotic device 125 encounters or is about to encounter an interior wall of the anatomical structure 107.

During the rotation, it is desirable for the point of view of the user 140 to have at least part of the ultrasound image of the robotic device 125 to appear to remain static in relation to the original reference image in the display on the GUI 145, in a three-dimensional volume or two-dimensional planes. This provides a stable visual reference for the rotation and the area of interest in the ultrasound image.

In case of a three-dimensional volume, the image processing/device tracking system 124 maintains a static ultrasound image orientation by changing the perspective of the three-dimensional model during the rotation, and adjusting any virtual cut-away planes.

In case of two-dimensional images (x-planes), the image acquisition by the imaging interface device 115 may be controlled to change with the rotation. For example, when using IMUs, the two-dimensional image plane B is virtually steered via electronic beam steering around the longitudinal axis 330 in the equal and opposite direction of the corresponding rotation of the imaging interface device 115, as measured by the IMUs. For example, when the IMUs sense 10 degrees of counterclockwise rotation, the image processing/device tracking system 124 causes the image plane B of the imaging interface device 115 to rotate 10 degrees clockwise. This keeps the image plane B in the same orientation relative to the anatomical structure 107 during the rotation. Alternatively, the image plane B can be anchored to the location of the robotic device 125 and/or an anatomical feature in the images while the imaging interface device 115 is manipulated, subject to constraints such that the image plane B rotates about the longitudinal axis 330, and intersects the transducer elements, and the image formation is possible using beam forming techniques.

Further in case of two-dimensional images, the imaging interface device 115 may be controlled to create arbitrary x-planes by selective activation and deactivation of different sets of ultrasound transducers/detecting elements in the transducer/detector array 222. Accordingly, as the imaging interface device 115 is rotated, the ultrasound system 110 activates the transducers and/or detecting elements of the imaging interface device 115 aligned with the initial position of the image plane B, and deactivates the remaining transducers and/or detecting elements, in order to keep the image plane B in the same orientation, thereby making the image plane B appear to be static relative to the anatomical structure 107.

The user 140 leaves the control mode and re-enters the imaging mode when the push button 216 is inactivated (e.g., released or depressed again). Then, the user 140 may again manipulate the imaging interface device 115 to obtain ultrasound images of the anatomical structure 107 and the robotic device 125, which are displayed on the GUI 145, from the new rotated orientation. In an embodiment, the imaging interface device 115 may continue to provide ultrasound images during the translation, regardless of the position of the push button 216, such that the movement of the robotic device 125 is captured and displayed on the GUI 145 along with the rotation of the ultrasound probe 215A.

Accordingly, the reference plane R may be used differently in the different rotation modes. In one rotation mode, the reference plane R is defined relative to arbitrary anatomy of the subject 105 for the purpose of rotation tracking, rotation controls articulation, or some other robotic or imaging parameter (in a continuous manner). In this scenario, the reference plane R is just a reference, and be substituted with IMU only. In another rotation mode, rotation of the imaging interface device 115 is used to place the reference plane R by rotation (e.g., about the distal tip 126 of the robotic device 125) in a continuous manner. Once placed relative to the anatomy of the subject 105, the new reference plane R may be used as a reference datum (travelling with the anatomy) for a subsequent servo loop controlled by the robot controller 122. For example, the robot controller 122 may articulate the robotic device 125 towards the new reference plane R. The image plane B in both rotation modes is used generally for user feedback. That is, depiction of the image plane B need not be provided, and if it is, the image plane B rotates in the opposite direction as the reference plane R. Or, the image plane B stays in the same plane relative to the anatomical structure 107 or robotic device 125 by direct tracking.

In addition to or instead of directional movement of the distal tip 126, the rotation input using the imaging interface device 115 may be detected and used to control other parameters associated with the imaging interface device 115 or the robotic device 125. For example, the rotation of the imaging interface device 115 may be used to control rotation of the robotic device 125 around its longitudinal axis. That is, the imaging processing/device tracking system 124 may be configured to interpret the rotation of the imaging interface device 115 around the longitudinal axis 330 as corresponding rotation of the robotic device 125 around its longitudinal axis, matching the angle (amount) and direction of rotation.

Also, the rotation of the imaging interface device 115 may be used to control various operations of the robotic device 125. For example, when the robotic device 125 comprises or controls a laser device, e.g., used for ablation or atherectomy procedures, the imaging processing/device tracking system 124 may be configured to interpret clockwise rotation of the imaging interface device 115 as an increase in laser power, and counterclockwise rotation as a decrease in laser power, or vice versa. The angle of rotation (clockwise or counterclockwise) may be interpreted as being proportional to the magnitude of the corresponding increase/decrease in laser power. Or, when the imaging interface device 115 is not rotating, translational movement of the imaging interface device 115 may control the desired speed setting of the laser. When the robotic device 125 comprises or controls a cutting tool, the imaging processing/device tracking system 124 may be configured to interpret clockwise rotation of the imaging interface device 115 as an increase in cutting speed or mechanical oscillation, and counterclockwise rotation as a decrease in cutting speed or mechanical oscillation, or vice versa. The angle of rotation may be interpreted as being proportional to the magnitude of the corresponding increase/decrease in cutting speed or mechanical oscillation. When the robotic device 125 comprises or controls a balloon catheter, the imaging processing/device tracking system 124 may be configured to interpret clockwise rotation of the imaging interface device 115 as an increase in balloon inflation rate, and counterclockwise rotation as a decrease in balloon inflation rate, or vice versa.

With regard to an atherectomy procedure in particular, the position of the imaging interface device 115 may control the orientation of the side-facing atherectomy cutter/laser of the robotic device 125 inside the anatomical structure 107. This may be done, for example, by positioning the imaging interface device 115 transversally on the leg of the subject 105. The robot controller 122 uses this orientation of the imaging interface device 115 to actuate the robotic device 125 to rotate/position the atherectomy body or cutting direction to align with the imaging interface device 115 as it is moved on the surface of the leg, thus facing the cutter towards the imaging interface device 115.

The rotation of the imaging interface device 115 may also be used to control displacement of a secondary device relative to the robotic device 125. For example, the robotic device 125 may be a catheter and the secondary device may be a guidewire within the catheter, in which case the imaging processing/device tracking system 124 may be configured to interpret clockwise rotation of the imaging interface device 115 as extending the secondary device, and counterclockwise rotation as retracting the secondary device, or vice versa. The angle of rotation may be interpreted as being proportional to the distance of the corresponding extension/retraction. Of course, the detected rotation may be used to adjust other parameters associated with the imaging interface device 115 and/or the robotic device 125 without departing from the scope of the present teachings.

In addition, the rotation of the imaging interface device 115 may be used to control operations of the ultrasound system 110, the imaging interface device 115 itself, and/or the GUI 145. For example, clockwise and counterclockwise rotation may be used to adjust image acquisition settings of the ultrasound system 110 or the imaging interface device 115, such as frequency, pulse wave, color, and power, for example. Likewise, the clockwise and counterclockwise rotation may be used to adjust display settings of the GUI 145. For example, the rotation may be used to control image contrast when displayed on the GUI 145. Or, for an image review using the GUI 145, the rotation may control a sequential scrolling through acquired ultrasound images.

In an embodiment, external tracking of the imaging interface device 115 and the robotic device 125 may be used to provide feedback, particularly when the robotic device 125 is not in the field of view of the ultrasound image or ultrasound volume. For example, one or more sensors may be provided on the imaging interface device 115 and/or the robotic device 125, so that their respective positions may be determined using electromagnetic (EM) tracking, regardless of whether the robotic device 125 is present in the field of view of the imaging interface device 115. Other examples of external tracking include optical tracking, acoustic tracking, electromagnetic tracking, ultrasound-sensed device tracking (e.g., Onvision^{®} Needle Tip Tracking), and IMU based tracking.

The imaging interface device 115 may be implemented using imaging modalities other than ultrasound, which is, however, not according to the claimed invention. For example, the imaging interface device 115 may provide single photon emission computed tomography (SPECT) imaging, x-ray imaging, or endoscopic/laparoscopic imaging. In this case, the ultrasound system 110 would be replaced by an imaging system compatible with the alternative imaging modality, as would be apparent to one skilled in the art.

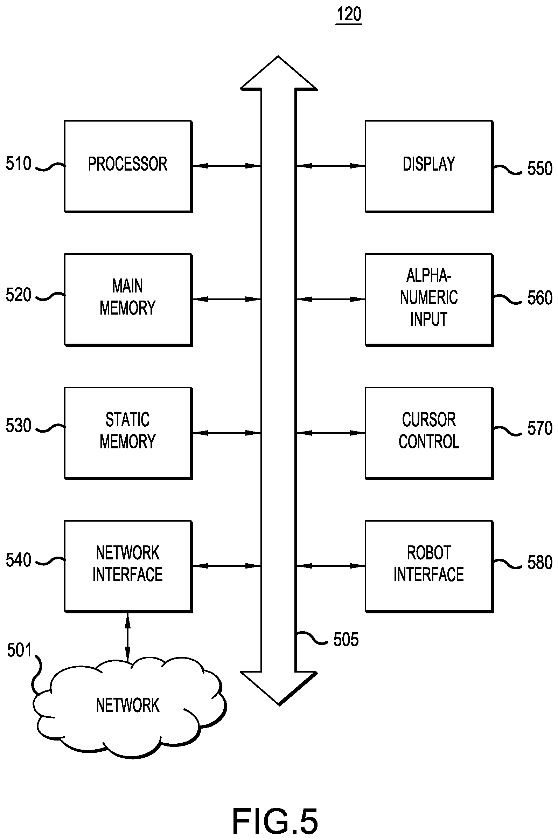

FIG. 5 is a simplified block diagram of a system controller for controlling imaging and a robotic device insertable into an anatomical structure of a subject, according to a representative embodiment.

Referring to FIG. 5, the system controller 120 includes software instructions that can be executed to perform any of the methods or computer-based functions disclosed herein. The system controller 120 may operate as a standalone device or may be connected, for example, using a network 501, to other computer systems or peripheral devices.

The system controller 120 includes a processor 510 configured to execute software instructions to perform functions as described in the various embodiments herein. The processor 510 is representative of one or more processing devices, and is configured to execute software instructions to perform functions as described in the various embodiments herein. The processor 510 may be implemented by field programmable gate arrays (FPGAs), application specific integrated circuits (ASICs), a general purpose computer, a central processing unit, a computer processor, a microprocessor, a microcontroller, a microcomputer, a state machine, programmable logic device, a processor chip, a digital signal processor (DSP), a graphics processing unit (GPU), or combinations thereof, using any combination of hardware, software, firmware, hard-wired logic circuits, or combinations thereof. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The system controller 120 further includes a main memory 520 and a static memory 530, where memories communicate with each other and the processor 510 via a bus 505. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time.

The main memory 520 and the static memory 530 are computer-readable mediums from which data and executable software instructions can be read by a computer (e.g., the processor 510). Each of the main memory 520 and the static memory 530 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memories may be volatile or non-volatile, secure and/or encrypted, unsecure and/or unencrypted. The main memory 520 and the static memory 530 may store various types of information, such as software algorithms, AI models including RNN and other neural network based models, and computer programs, all of which are executable by the processor 510.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the system controller 120 further includes a display 550, such as a liquid crystal display (LCD), an organic light emitting diode (OLED), a flat panel display, a solid-state display, or a cathode ray tube (CRT), for example. The GUI 145 may be incorporated into the display 550. Additionally, the system controller 120 includes an input device 560, such as a keyboard/virtual keyboard or touch-sensitive input screen or speech input with speech recognition, and a cursor control device 570, such as a mouse or touch-sensitive input screen or pad. The system controller also includes a robot interface 580 configured to control movement of the robotic device 125 in accordance with the embodiments described herein.

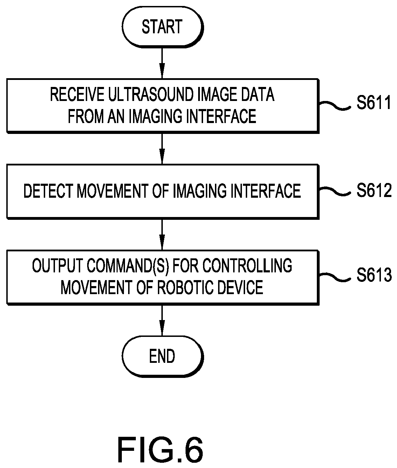

FIG. 6 is a flow diagram of a method for controlling imaging and a robotic device insertable into an anatomical structure of a subject, according to a representative embodiment. The various steps of the flow diagram may be performed by the system controller 120, discussed above, for example.

Referring to FIG. 6, ultrasound image data is received from an imaging interface device (e.g., imaging interface device 115) in block S611. The ultrasound image data corresponds to an ultrasound image showing an anatomical structure (e.g., anatomical structure 107) in the subject and a portion of a robotic device (e.g., robotic device 125) within in the anatomical structure. The imaging interface device is maneuverable for providing the ultrasound image data. For example, the imaging interface device may be an ultrasound probe that is maneuverable by a user to obtain various images of the anatomical structure and the robotic device during an interventional procedure, where the images are viewable on a display (e.g., GUI 145). The user may maneuver the ultrasound probe directly by making adjustments while holding the ultrasound probe, or remotely though a robotic interface, to obtain the desired ultrasound image.

In an embodiment, the imaging interface device has a built-in actuator enabling the user to select an imaging mode and a control mode of operation. In the imaging mode, image data received from the imaging interface device is processed to provide images of the anatomical structure and the portion of the robotic device within the anatomical structure. In the control mode, information indicative of translational and/or rotational movement of the imaging interface device on the surface of the subject received from the imaging interface device is processed to control movement of robotic device within the anatomical in association with the translation and/or rotational movement of the imaging interface device. When the imaging interface device is an ultrasound probe, the actuator may comprise one or more push buttons, slider buttons and/or touch sensitive buttons conveniently located on the handle of the ultrasound probe, as discussed above. This enables the user to select between the imaging and control modes with one hand, while holding the ultrasound probe, avoiding interruption of the interventional procedure and eliminating the need for another person to separately operate the robotic device (or the imaging device).

In block S612, a movement of the imaging interface device is detected. As discussed above, the movement of the imaging interface device may be a translational movement or a rotational movement, and is detectable in the control mode of operation. Translational movement may be detected based on direction, distance and/or velocity data received from IMU(s) on the imaging interface device. Rotational movement may be detected based on position data received from the IMU(s) on the imaging interface device, or may be detected by direct image observation using image processing, such as speckle tracking processing, for example.

In block S613, at least one command is output for controlling movement of the robotic device from a first position to a second position in response to the detected movement of the imaging interface device. The at least one command may be provided to a robot controller (e.g., a PID controller), for example, which in turn controls servos to move the robotic device in association with the detected movement of the imaging interface device. Alternatively the command may be stored in a non-transitory memory, for a delayed use or additional processing (e.g. formatting, additional check for debugging purpose..), before any use by the robot controller.

When the movement of the imaging interface device is a translational movement, the method includes defining an image plane B and a reference plane R in the ultrasound image relative to the imaging interface device. The image plane B extends from the imaging interface device into the subject, and includes a longitudinal axis of the robotic device and/or a longitudinal axis or other features of the anatomical structure. The reference plane R intersects the image plane B at the first position of the robotic device. More particularly, the robotic device includes a tracking feature, such as the distal tip of the robotic device, a bend or a marker, for example, which defines the first position, and the reference plane R intersects the image plane B at this tracking feature. The reference plane R is translated in association with the translational movement of the imaging interface device, and at least one command for controlling the robotic device is output to the robot controller based on a distance between the trackable feature of the robotic device the translated reference plane R. In an embodiment, the robotic device may be controlled based on the distance between the trackable feature of the robotic device and the translated reference plane R, where the robotic device is servoed toward the reference plane R being translated using the distance as a position error. That is, the robot controller attempts to correct for the position error by reducing the distance between the trackable feature and the reference plane R.

When the movement of the imaging interface device is a rotational movement, the method includes defining an image plane B in the ultrasound image relative to the imaging interface device, the image plane B and a reference plane R in the ultrasound image relative to the imaging interface device. The image plane B extends from the imaging interface device into the subject, and includes a longitudinal axis of the robotic device. The reference plane R is defined in the ultrasound image relative to the imaging interface device, intersecting the image plane B at the first position of the robotic device. A direction and angle of the rotational movement of the imaging interface device are detected, e.g., from direct image observation using image processing, or position data provided by IMU(s) on the imaging interface device. The reference plane R is rotated in the same direction and angle as the rotational movement of the imaging interface device. At least one command for controlling the robotic device is output to the robot controller in response to the rotated reference plane R.

With regard to the rotational movement, it is desirable to maintain the image plane B in in a constant image plane as viewed on the display. For a three-dimensional volume, the image plane B remains visually static during the rotation by changing the perspective of the three-dimensional model and adjusting any virtual cut-away planes. For two-dimensional images, the image plane B may remain visually static during the rotation by virtually steering the image plane B around a longitudinal axis of the imaging interface device in the opposite direction as the corresponding rotation, using position data measured by one or more IMUs. Also for two-dimensional images, the image plane B may remain visually static during the rotation by causing the imaging interface device to selectively activate ultrasound transducers and/or detecting elements in the imaging interface device to maintain the ultrasound image in a constant image plane.

**In** accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although developing adaptable predictive analytics has been described with reference to several exemplary embodiments, it is understood that the words that have been used are words of description and illustration, rather than words of limitation. Changes may be made within the purview of the appended claims, as presently stated and as amended, without departing from the scope and spirit of interventional procedure optimization in its aspects. Although developing adaptable predictive analytics has been described with reference to particular means, materials and embodiments, developing adaptable predictive analytics is not intended to be limited to the particulars disclosed; rather developing adaptable predictive analytics extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept.

## Claims

1. A controller for controlling a robotic device (125) configured for insertion into an anatomical structure (106) of a subject (105), the controller comprising:
at least one processor (510); and
a non-transitory memory for storing machine executable instructions that, when executed by the at least one processor (510), cause the at least one processor (510) to:
receive ultrasound image data from an imaging interface device (115), the ultrasound image data corresponding to an ultrasound image showing the anatomical structure (106) and a portion of the robotic device (125) within in the anatomical structure (106), wherein the imaging interface device (115) is maneuverable for providing the ultrasound image data;
detect a movement, or receive an information representative of the detected movement of the imaging interface device (115) derived from content of the ultrasound image data; and
output at least one command for controlling movement of the robotic device (125) from a first position to a second position in response to the detected movement of the imaging interface device (115).

2. The controller of claim 1, wherein the movement of the imaging interface device (115) comprises at least one of a translational movement or a rotational movement.

3. The controller of claim 2, wherein the movement of the imaging interface device (115) is a translational movement, and outputting the at least one command for controlling the movement of the robotic device (125) comprises:
defining an image plane B in the ultrasound image relative to the imaging interface device (115), the image plane B including a longitudinal axis (330) of the robotic device (125);
defining a reference plane R in the ultrasound image relative to the imaging interface device (115) that intersects the image plane B at the first position of the robotic device (125), wherein the reference plane R moves with the imaging interface device (115); and
outputting the at least one command for controlling the robotic device (125) based on a distance between a trackable feature of the robotic device (125) and the translated reference plane R.

4. The controller of claim 3, wherein controlling the robotic device (125) based on the distance between the trackable feature of the robotic device (125) and the translated reference plane R comprises:
causing the robotic device (125) to be served toward the translated reference plane using the distance as a position error.

5. The controller of claim 4, wherein the trackable feature of the robotic device (125) comprises one of a distal end of the robotic device (125), a marker on the rotational device, a predetermined section of the robotic device (125), or a bend in the robotic device (125).

6. The controller of claim 2, wherein the movement of the imaging interface device (115) is a rotational movement, and outputting the at least one command for controlling the movement of the robotic device (125) comprises:
defining a reference plane R relative in the ultrasound image relative to the imaging interface device (115) that is referenced to anatomical features;
detecting a direction, or receiving an information representative of a direction and angle of the rotational movement of the imaging interface device (115); and
outputting the at least one command for controlling the robotic device (125) in response to an amount the reference plane R rotates relative to the imaging interface device (115).

7. The controller of claim 6, wherein the reference plane R is rotated in accordance with one or more boundary conditions limiting a range of rotation of the reference plane R.

8. The controller of claim 6, wherein the machine executable instructions further cause the at least one processor (510) to:
selectively activate ultrasound transducers of a transducer array and/or detect elements in the imaging interface device (115) to maintain the ultrasound image in a constant image plane B during the rotational movement of the imaging interface device (115).

9. The controller of claim 6, wherein outputting the at least one command for controlling the robotic device (125) in response to the rotated reference plane R comprises causing the robotic device (125) to move in the direction and the angle of the rotational movement associated with the rotated reference plane R.

10. The controller of claim 6, wherein outputting the at least one command for controlling the robotic device (125) in response to the rotated reference plane R comprises causing the robotic device (125) to rotate along a longitudinal axis (330) in the direction and the angle of the rotational movement associated with the rotated reference plane R.

11. The controller of claim 6, wherein outputting the at least one command for controlling the robotic device (125) in response to the rotated reference plane R comprises causing deployment or activation of a surgical tool or instrument to be triggered.

12. The controller of claim 1, wherein the imaging interface device (115) is selectively operable in an imaging mode and one or more control modes, wherein the at least one processor (510) receives the ultrasound image data from the imaging interface device (115) in the imaging mode, and detects the movement, or receives information representative of the movement of the imaging interface device (115) in the control mode, the controller being optionally configured to select the imaging mode and the control mode.

13. A system for controlling a robotic device (125) configured for insertion into an anatomical structure (106) of a subject (105), comprising:
- the controller of claim 12;
- a movable imaging interface device (115) arranged to send ultrasound image data to the controller, the ultrasound image data corresponding to an ultrasound image showing the anatomical structure (106) and a portion of the robotic device (125) within in the anatomical structure (106), and wherein the imaging interface device (115) comprises an actuator operable by the user (140) while holding imagining interface device to interactively select the imaging mode and the control mode.

14. The system of claim 14, wherein the imaging interface device (115) comprises an ultrasound probe (215A), and the actuator comprises at least one of a push button (216), a slider button (217) or a touch sensitive button on a probe handle (221) of the ultrasound probe (215A).

15. A non-transitory computer readable medium storing instructions for controlling a robotic device (125) configured for insertion into an anatomical structure (106) of a subject (105) that, when executed by at least one processor (510), cause the at least one processor (510) to:
receive ultrasound image data from an imaging interface device (115), the ultrasound image data corresponding to an ultrasound image showing the anatomical structure (106) and a portion of the robotic device (125) within in the anatomical structure (106), wherein the imaging interface device (115) is maneuverable for providing the ultrasound image data;
detect a movement, or receive an information representative of the detected movement of the imaging interface device (115) derived from content of the ultrasound image data; and
output at least one command for controlling movement of the robotic device (125) from a first position to the second position in response to the detected movement of the imaging interface device (115).

## Patentansprüche

1. Steuereinheit zum Steuern einer Robotervorrichtung (125), die zur Einfuhr in eine anatomische Struktur (106) eines Subjekts (105) konfiguriert ist, wobei die Steuereinheit Folgendes umfasst:
mindestens einen Prozessor (510); und
einen nichtflüchtigen Speicher zum Speichern maschinenausführbarer Anweisungen, die, wenn sie von dem mindestens einen Prozessor (510) ausgeführt werden, den mindestens einen Prozessor (510) zu Folgendem veranlassen:
Empfangen von Ultraschallbilddaten von einer Bildgebungsschnittstellenvorrichtung (115), wobei die Ultraschallbilddaten einem Ultraschallbild entsprechen, das die anatomische Struktur (106) und einen Teil der Robotervorrichtung (125) innerhalb der anatomischen Struktur (106) zeigt, wobei die Bildgebungsschnittstellenvorrichtung (115) zum Bereitstellen der Ultraschallbilddaten manövrierbar ist;
Erfassen einer Bewegung oder Empfangen von Informationen, welche die erfasste Bewegung der Bildgebungsschnittstellenvorrichtung (115) darstellen, wobei sie aus dem Inhalt der Ultraschallbilddaten abgeleitet sind; und
Ausgeben mindestens eines Befehls zum Steuern der Bewegung der Robotervorrichtung (125) von einer ersten Position zu einer zweiten Position als Reaktion auf die erfasste Bewegung der Bildschnittstellenvorrichtung (115).

2. Steuereinheit nach Anspruch 1, wobei die Bewegung der Bildschnittstellenvorrichtung (115) mindestens eine von einer Translationsbewegung oder einer Drehbewegung umfasst.

3. Steuereinheit nach Anspruch 2, wobei die Bewegung der Bildschnittstellenvorrichtung (115) eine Translationsbewegung ist und das Ausgeben des mindestens einen Befehls zum Steuern der Bewegung der Robotervorrichtung (125) Folgendes umfasst:
Definieren einer Bildebene B in dem Ultraschallbild relativ zu der Bildgebungsschnittstellenvorrichtung (115), wobei die Bildebene B eine Längsachse (330) der Robotervorrichtung (125) beinhaltet;
Definieren einer Referenzebene R in dem Ultraschallbild relativ zu der Bildgebungsschnittstellenvorrichtung (115), die die Bildebene B an der ersten Position der Robotervorrichtung (125) schneidet, wobei sich die Referenzebene R mit der Bildgebungsschnittstellenvorrichtung (115) bewegt; und
Ausgeben des mindestens einen Befehls zum Steuern der Robotervorrichtung (125) basierend auf einer Distanz zwischen einem verfolgbaren Merkmal der Robotervorrichtung (125) und der verschobenen Referenzebene R.

4. Steuereinheit nach Anspruch 3, wobei das Steuern der Robotervorrichtung (125) basierend auf der Distanz zwischen dem verfolgbaren Merkmal der Robotervorrichtung (125) und der verschobenen Referenzebene R Folgendes umfasst:
Veranlassen der Robotervorrichtung (125), in Richtung der verschobenen Referenzebene unter Verwendung der Distanz als einen Positionsfehler bedient zu werden.

5. Steuereinheit nach Anspruch 4, wobei das verfolgbare Merkmal der Robotervorrichtung (125) eines von einem distalen Ende der Robotervorrichtung (125), einer Markierung auf der Drehvorrichtung, einem vorbestimmten Abschnitt der Robotervorrichtung (125) oder einer Biegung in der Robotervorrichtung (125) umfasst.

6. Steuereinheit nach Anspruch 2, wobei die Bewegung der Bildschnittstellenvorrichtung (115) eine Drehbewegung ist und das Ausgeben des mindestens einen Befehls zum Steuern der Bewegung der Robotervorrichtung (125) Folgendes umfasst:
Definieren einer Referenzebene R relativ in dem Ultraschallbild relativ zu der Bildgebungsschnittstellenvorrichtung (115), die sich auf anatomische Merkmale bezieht;
Erfassen einer Richtung oder Empfangen von Informationen, die eine Richtung und einen Winkel der Drehbewegung der Bildschnittstellenvorrichtung (115) darstellen; und
Ausgeben des mindestens einen Befehls zum Steuern der Robotervorrichtung (125) als Reaktion auf einen Betrag, um den sich die Referenzebene R relativ zu der Bildschnittstellenvorrichtung (115) dreht.

7. Steuereinheit nach Anspruch 6, wobei die Referenzebene R in Übereinstimmung mit einer oder mehreren Randbedingungen gedreht wird, die einen Drehbereich der Referenzebene R begrenzen.

8. Steuereinheit nach Anspruch 6, wobei die maschinenausführbaren Anweisungen den mindestens einen Prozessor (510) weiter zu Folgendem veranlassen:
selektives Aktivieren von Ultraschallwandlern eines Wandlerarrays und/oder Erfassungselementen in der Bildschnittstellenvorrichtung (115), um das Ultraschallbild während der Drehbewegung der Bildschnittstellenvorrichtung (115) in einer konstanten Bildebene B zu halten.

9. Steuereinheit nach Anspruch 6, wobei das Ausgeben des mindestens einen Befehls zum Steuern der Robotervorrichtung (125) als Reaktion auf die gedrehte Referenzebene R umfasst, die Robotervorrichtung (125) zu veranlassen, sich in der Richtung und dem Winkel der Drehbewegung zu bewegen, die der gedrehten Referenzebene R zugeordnet ist.

10. Steuereinheit nach Anspruch 6, wobei das Ausgeben des mindestens einen Befehls zum Steuern der Robotervorrichtung (125) als Reaktion auf die gedrehte Referenzebene R umfasst, die Robotervorrichtung (125) zu veranlassen, sich entlang einer Längsachse (330) in der Richtung und dem Winkel der Drehbewegung zu drehen, die der gedrehten Referenzebene R zugeordnet ist.

11. Steuereinheit nach Anspruch 6, wobei das Ausgeben des mindestens einen Befehls zum Steuern der Robotervorrichtung (125) als Reaktion auf die gedrehte Referenzebene R umfasst zu veranlassen, dass Einsatz oder Aktivierung eines chirurgischen Werkzeugs oder Instruments ausgelöst wird.

12. Steuereinheit nach Anspruch 1, wobei die Bildgebungsschnittstellenvorrichtung (115) wahlweise in einem Bildgebungsmodus und einem oder mehreren Steuermodi betrieben werden kann, wobei der mindestens eine Prozessor (510) in dem Bildgebungsmodus die Ultraschallbilddaten von der Bildgebungsschnittstellenvorrichtung (115) empfängt und die Bewegung erfasst, oder in dem Steuermodus Informationen empfängt, die die Bewegung der Bildgebungsschnittstellenvorrichtung (115) darstellen, wobei die Steuereinheit optional dazu konfiguriert ist, den Bildgebungsmodus und den Steuermodus auszuwählen.

13. System zum Steuern einer Robotervorrichtung (125), die zur Einfuhr in eine anatomische Struktur (106) eines Subjekts (105) konfiguriert ist, das Folgendes umfasst:
- die Steuereinheit nach Anspruch 12;
- eine bewegliche Bildgebungsschnittstellenvorrichtung (115), die dazu eingerichtet ist, Ultraschallbilddaten an die Steuereinheit zu senden, wobei die Ultraschallbilddaten einem Ultraschallbild entsprechen, das die anatomische Struktur (106) und einen Teil der Robotervorrichtung (125) innerhalb der anatomischen Struktur (106) zeigt, und wobei die Bildgebungsschnittstellenvorrichtung (115) eine Betätigungsvorrichtung umfasst, die von dem Benutzer (140) bedient werden kann, während er die Bildgebungsschnittstellenvorrichtung hält, um interaktiv den Bildgebungsmodus und den Steuerungsmodus auszuwählen.

14. System nach Anspruch 14, wobei die Bildgebungsschnittstellenvorrichtung (115) eine Ultraschallsonde (215 A) umfasst und die Betätigungsvorrichtung mindestens einen Druckknopf (216), einen Schiebeknopf (217) oder einen berührungsempfindlichen Knopf an einem Sondengriff (221) der Ultraschallsonde (215A) umfasst.

15. Nichtflüchtiges computerlesbares Medium, das Anweisungen zum Steuern einer Robotervorrichtung (125) speichert, die zur Einfuhr in eine anatomische Struktur (106) eines Subjekts (105) konfiguriert ist, die, wenn sie von mindestens einem Prozessor (510) ausgeführt werden, den mindestens einen Prozessor (510) zu Folgendem veranlassen:
Empfangen von Ultraschallbilddaten von einer Bildgebungsschnittstellenvorrichtung (115), wobei die Ultraschallbilddaten einem Ultraschallbild entsprechen, das die anatomische Struktur (106) und einen Teil der Robotervorrichtung (125) innerhalb der anatomischen Struktur (106) zeigt, wobei die Bildgebungsschnittstellenvorrichtung (115) zum Bereitstellen der Ultraschallbilddaten manövrierbar ist;
Erfassen einer Bewegung oder Empfangen von Informationen, welche die erfasste Bewegung der Bildgebungsschnittstellenvorrichtung (115) darstellen, wobei sie aus dem Inhalt der Ultraschallbilddaten abgeleitet sind; und
Ausgeben mindestens eines Befehls zum Steuern der Bewegung der Robotervorrichtung (125) von einer ersten Position zu der zweiten Position als Reaktion auf die erfasste Bewegung der Bildschnittstellenvorrichtung (115).

## Revendications

1. Dispositif de commande pour commander un dispositif robotique (125) configuré pour être inséré dans une structure anatomique (106) d'un sujet (105), le dispositif de commande comprenant :
au moins un processeur (510) ; et
une mémoire non transitoire pour stocker des instructions exécutables par machine qui, lorsqu'elles sont exécutées par le au moins un processeur (510), amènent le au moins un processeur (510) à :
recevoir des données d'image ultrasonore à partir d'un dispositif d'interface d'imagerie (115), les données d'image ultrasonore correspondant à une image ultrasonore montrant la structure anatomique (106) et une partie du dispositif robotique (125) au sein de la structure anatomique (106), dans lequel le dispositif d'interface d'imagerie (115) est manœuvrable pour fournir les données d'image ultrasonore ;
détecter un mouvement, ou recevoir une information représentative du mouvement détecté du dispositif d'interface d'imagerie (115) déduite du contenu des données d'image ultrasonore ; et
émettre au moins une commande pour commander le mouvement du dispositif robotique (125) d'une première position à une seconde position en réponse au mouvement détecté du dispositif d'interface d'imagerie (115).

2. Dispositif de commande selon la revendication 1, dans lequel le mouvement du dispositif d'interface d'imagerie (115) comprend au moins l'un d'un mouvement de translation ou d'un mouvement de rotation.

3. Dispositif de commande selon la revendication 2, dans lequel le mouvement du dispositif d'interface d'imagerie (115) est un mouvement de translation, et l'émission de la au moins une commande pour commander le mouvement du dispositif robotique (125) comprend :
la définition d'un plan d'image B dans l'image ultrasonore par rapport au dispositif d'interface d'imagerie (115), le plan d'image B incluant un axe longitudinal (330) du dispositif robotique (125) ;
la définition d'un plan de référence R dans l'image ultrasonore par rapport au dispositif d'interface d'imagerie (115) qui coupe le plan d'image B à la première position du dispositif robotique (125), dans lequel le plan de référence R se déplace avec le dispositif d'interface d'imagerie (115) ; et
l'émission de la au moins une commande pour commander le dispositif robotique (125) sur la base d'une distance entre une caractéristique pouvant être suivie du dispositif robotique (125) et le plan de référence R translaté.

4. Dispositif de commande selon la revendication 3, dans lequel la commande du dispositif robotique (125) sur la base de la distance entre la caractéristique pouvant être suivie du dispositif robotique (125) et le plan de référence R translaté comprend :
le fait d'amener le dispositif robotique (125) à être asservi vers le plan de référence translaté en utilisant la distance comme erreur de position.

5. Dispositif de commande selon la revendication 4, dans lequel la caractéristique pouvant être suivie du dispositif robotique (125) comprend un parmi une extrémité distale du dispositif robotique (125), un marqueur sur le dispositif de rotation, une section prédéterminée du dispositif robotique (125) ou un coude dans le dispositif robotique (125).

6. Dispositif de commande selon la revendication 2, dans lequel le mouvement du dispositif d'interface d'imagerie (115) est un mouvement de rotation, et l'émission de la au moins une commande pour commander le mouvement du dispositif robotique (125) comprend :
la définition d'un plan de référence R relatif dans l'image ultrasonore par rapport au dispositif d'interface d'imagerie (115) qui est rapporté à des caractéristiques anatomiques ;
la détection d'une direction ou la réception d'une information représentative d'une direction et d'un angle du mouvement de rotation du dispositif d'interface d'imagerie (115) ; et
l'émission de la au moins une commande pour commander le dispositif robotique (125) en réponse à une quantité de rotation du plan de référence R par rapport au dispositif d'interface d'imagerie (115).

7. Dispositif de commande selon la revendication 6, dans lequel le plan de référence R est tourné selon une ou plusieurs conditions limites limitant une plage de rotation du plan de référence R.

8. Dispositif de commande selon la revendication 6, dans lequel les instructions exécutables par machine amènent en outre le au moins un processeur (510) à :
activer sélectivement des transducteurs à ultrasons d'un réseau de transducteurs et/ou détecter des éléments dans le dispositif d'interface d'imagerie (115) pour maintenir l'image ultrasonore dans un plan d'image B constant pendant le mouvement de rotation du dispositif d'interface d'imagerie (115).

9. Dispositif de commande selon la revendication 6, dans lequel l'émission de la au moins une commande pour commander le dispositif robotique (125) en réponse au plan de référence R tourné comprend le fait d'amener le dispositif robotique (125) à se déplacer dans la direction et l'angle du mouvement de rotation associé au plan de référence R tourné.

10. Dispositif de commande selon la revendication 6, dans lequel l'émission de la au moins une commande pour commander le dispositif robotique (125) en réponse au plan de référence R tourné comprend le fait d'amener le dispositif robotique (125) à tourner le long d'un axe longitudinal (330) dans la direction et l'angle du mouvement de rotation associé au plan de référence R tourné.

11. Dispositif de commande selon la revendication 6, dans lequel l'émission de la au moins une commande pour commander le dispositif robotique (125) en réponse au plan de référence R tourné comprend le fait de provoquer le déclenchement du déploiement ou de l'activation d'un outil ou d'un instrument chirurgical.

12. Dispositif de commande selon la revendication 1, dans lequel le dispositif d'interface d'imagerie (115) peut être utilisé sélectivement dans un mode d'imagerie et un ou plusieurs modes de commande, dans lequel le au moins un processeur (510) reçoit les données d'image ultrasonore en provenance du dispositif d'interface d'imagerie (115) dans le mode d'imagerie, et détecte le mouvement, ou reçoit des informations représentatives du mouvement du dispositif d'interface d'imagerie (115) dans le mode de commande, le dispositif de commande étant facultativement configuré pour sélectionner le mode d'imagerie et le mode de commande.

13. Système pour commander un dispositif robotique (125) configuré pour être inséré dans une structure anatomique (106) d'un sujet (105), comprenant :
- le dispositif de commande selon la revendication 12 ;
- un dispositif d'interface d'imagerie (115) mobile conçu pour envoyer des données d'image ultrasonore au dispositif de commande, les données d'image ultrasonore correspondant à une image ultrasonore montrant la structure anatomique (106) et une partie du dispositif robotique (125) au sein de la structure anatomique (106), et dans lequel le dispositif d'interface d'imagerie (115) comprend un actionneur pouvant être utilisé par l'utilisateur (140) tout en tenant le dispositif d'interface d'imagerie pour sélectionner de manière interactive le mode d'imagerie et le mode de commande.

14. Système selon la revendication 14, dans lequel le dispositif d'interface d'imagerie (115) comprend une sonde à ultrasons (215A), et l'actionneur comprend au moins un parmi un bouton-poussoir (216), un bouton coulissant (217) ou un bouton tactile sur un manche (221) de sonde de la sonde à ultrasons (215A).

15. Support non transitoire lisible par ordinateur stockant des instructions pour commander un dispositif robotique (125) configuré pour être inséré dans une structure anatomique (106) d'un sujet (105) qui, lorsqu'elles sont exécutée par au moins un processeur (510), amènent le au moins un processeur (510) à :
recevoir des données d'image ultrasonore à partir d'un dispositif d'interface d'imagerie (115), les données d'image ultrasonore correspondant à une image ultrasonore montrant la structure anatomique (106) et une partie du dispositif robotique (125) au sein de la structure anatomique (106), dans lequel le dispositif d'interface d'imagerie (115) est manœuvrable pour fournir les données d'image ultrasonore ;
détecter un mouvement, ou recevoir une information représentative du mouvement détecté du dispositif d'interface d'imagerie (115) déduite du contenu des données d'image ultrasonore ; et
émettre au moins une commande pour commander le mouvement du dispositif robotique (125) d'une première position à la seconde position en réponse au mouvement détecté du dispositif d'interface d'imagerie (115).
